# EUROPÄISCHE PATENTSCHRIFT

(11) **EP 0 931 523 B1**
(45) Veröffentlichungstag und Bekanntmachung des Hinweises auf die Patenterteilung: **25.06.2003**
(21) Anmeldenummer: 98810030.1
(22) Anmeldetag: 22.01.1998
(51) Int. Cl.: A61F 2/46

(54) **System aus einer künstlichen Gelenkkomponente und einem Instrument zur Handhabung der künstlichen Gelenkkomponente**
System having an artificial joint component and an instrument for handling the artificial joint component
Système comportant un élément articulaire artificiel et un instrument pour la manutention du élément articulaire artificiel

(43) Veröffentlichungstag der Anmeldung: 28.07.1999
(73) Patentinhaber: Centerpulse Orthopedics Ltd., 6340 Baar (CH)
(72) Erfinder: Willi, Thomas, 8405 Winterthur (CH); Wymann, Burkhard, 8353 Elgg (CH)
(74) Vertreter: Manitz, Finsterwald & Partner Gbr

(56) Entgegenhaltungen:
- EP-A- 0 190 982
- EP-A- 0 453 694
- WO-A-94/21199
- WO-A-97/42915
- FR-A- 2 710 522
- GB-A- 2 299 758
- SU-A- 1 680 149
- US-A- 5 540 697

## Beschreibung

Die Erfindung betrifft ein System mit einer künstlichen Gelenkkomponente, insbesondere einer künstlichen Hüftgelenkpfanne, und einem Instrument zur Handhabung der Künstlichen Gelenkkomponente gemäss dem Oberbegriff des unabhängigen Patentanspruchs. Ein solches System ist aus GB-A-2 299 758 bekannt.

Derartige Handhabungsinstrumente - unter Handhabung wird insbesondere auch das Ein- bzw. Herausdrehen verstanden - kommen ganz allgemein in der Gelenkchirurgie zum Einsatz, speziell aber in der Hüftgelenkchirurgie. So muss z.B. bei der Implantation von künstlichen Hüftgelenkpfannen die künstliche Hüftgelenkpfanne im Operationssaal für den Chirurgen bzw. für den Orthopäden einfach und zuverlässig handhabbar sein. Bekannte Handhabungsinstrumente kommen beispielsweise derart zum Einsatz, dass bei einem bereits zur Aufnahme der künstlichen Hüftgelenkpfanne präparierten Beckenknochen zunächst die Hüftgelenkpfanne in einer an dem Instrument vorgesehenen Greifeinrichtung quasi eingespannt wird. Anschliessend wird die Pfanne an die gewünschte Stelle in den Beckenknochen eingebracht. Speziell bei solchen Hüftgelenkpfannen, die auf ihrer Aussenschale ein Gewinde zum Eindrehen in das Knochengewebe aufweisen - oder besser gesagt einzelne Stücke eines Gewindes, da ja beim Eindrehen der Gewindestücke in das Knochengewebe das dabei herausgetrennte Knochengewebe auch abtransportiert werden muss - muss das Handhabungsinstrument mit der eingespannten Hüftgelenkpfanne noch mit einem geeigneten Eindrehinstrument (z.B. einer Ratsche) verbunden werden, damit der Chirurg bzw. Orthopäde die zum Eindrehen der Hüftgelenkpfanne erforderliche Kraft (bzw. das erforderliche Drehmoment) auf die Pfanne übertragen kann. Ähnliches gilt für das Herausdrehen einer Pfanne, sollte es erforderlich sein, eine zu einem früheren Zeitpunkt implantierte Hüftgelenkpfanne entfernen zu müssen.

Ein bekanntes Handhabungsintrument dieser Art weist einen hohlzylindrischen Stab auf, an dessen distalem Ende eine Greifeinrichtung angeordnet ist, die drei in Umfangsrichtung gleichmässig voneinander beabstandete, z.B. schwenkbar gelagerte, Greifbacken umfasst. An der Aussenwand der Gelenkpfanne sind in Bezug auf die Rotationsachse der Pfanne (die beim Einspannen der Pfanne mit der Längsachse des Stabs zusammenfällt) zylindrische Kontaktflächen vorgesehen, die beim Einspannen der Pfanne mit entsprechend zylindrisch in Bezug auf die Längsachse des Instruments angeordneten, nach innen weisenden Flächen an den Greifbacken zusammenwirken. Ist die Pfanne zwischen den Greifbacken eingespannt, so kann das Handhabungsinstrument mit der Ratsche verbunden werden und anschliessend die Pfanne in den Beckenknochen eingedreht werden.

Dieses bekannte Instrument ist grundsätzlich funktionstüchtig, weist aber noch gewisse Verbesserungsmöglichkeiten auf. So kommt es gelegentlich vor, dass bei der Übertragung von sehr grossen Kräften (bzw. Momenten) beim Eindrehen der Pfanne bei einem leichten Verkippen der aus dem Handhabungsinstrument und der Ratsche bestehenden Eindrehvorrichtung die Greifbacken von den Kontaktflächen auf der Aussenwand der Aussenschale der Hüftgelenkpfanne abrutschen. Das Handhabungsinstrument muss dann erneut mit der Pfanne verbunden werden, die Pfanne muss also erneut zwischen den Greifbacken eingespannt werden, bevor der Eindrehvorgang fortgesetzt werden kann. Dies ist während einer Operation ausserordentlich unerwünscht, vor allem dann, wenn die Pfanne bereits zu einem Teil in den vorbereiteten Beckenknochen eingedreht ist und somit das Handhabungsinstrument mit der bereits teilweise eingedrehten Pfanne verbunden werden muss.

Ausserdem lassen sich mit diesem bekannten Instrument Kräfte (bzw. Momente) einer Grösse, wie sie beim Herausdrehen einer zu einem früheren Zeitpunkt implantierten Hüftgelenkpfanne erforderlich sind, praktisch nicht übertragen, weil zum Herausdrehen einer bereits in das Knochengewebe "eingewachsenen" Pfanne deutlich grössere Kräfte (bzw. Momente) erforderlich sind als zum Eindrehen einer Pfanne.

Es ist daher Aufgabe der Erfindung, ein Handhabungsinstrument für eine künstliche Gelenkkomponente, insbesondere eine künstliche Hüftgelenkpfanne, vorzuschlagen, welches die vorstehend genannten Nachteile nicht aufweist und welches insbesondere beim Verkippen des Instruments nicht von den Kontaktflächen auf der Aussendwand der Pfanne abrutscht und welches insbesondere zum Eindrehen als auch zum Herausdrehen einer künstlichen Hüftgelenkpfanne geeignet ist.

Diese Aufgabe wird erfindungsgemäss durch ein Handhabungsinstrument mit den Merkmalen des unabhängigen Patentanspruchs gelöst. Dabei ist an den Greifbacken jeweils eine konische Fläche vorgesehen, die zum Greifen der Gelenkkomponente mit einer an der Gelenkkomponente entsprechend vorgesehenen konischen Fläche in Eingriff bringbar ist. Nach dem Einspannen der Komponente zwischen den - vorzugsweise schwenkbar gelagerten - Greifbacken kann wegen des Eingriffs der konischen Flächen miteinander (Hinterschneidung) kein Abrutschen des Instruments mehr erfolgen. Ausserdem können mittels des Eingriffs der konischen Flächen auch sehr viel grössere Kräfte (bzw. Momente) auf die Komponente, z.B. auf die bereits erwähnte künstliche Hüftgelenkpfanne, übertragen werden, wodurch das Instrument sowohl zum Eindrehen einer neuen Hüftgelenkpfanne, aber auch zum Herausdrehen einer bereits zu einem früheren Zeitpunkt implantierten Pfanne geeignet ist.

Prinzipiell kann die jeweilge konische Fläche sowohl an der Innenwand der Gelenkkomponente als auch an der Aussenwand vorgesehen sein. Vorzugsweise ist sie aber auf der Aussenwand der Gelenkkomponente, insbesondere auf der Aussenwand der Aussenschale einer Hüftgelenkpfanne, vorgesehen und läuft in distaler Richtung betrachtet nach innen zu. Entsprechend ist bei einem Ausführungsbeispiel des erfindungsgemässen Handhabungsinstruments die entsprechende konische Fläche auf der Innenseite der jeweiligen Greifbacke vorgesehen und ist ebenfalls in distaler Richtung betrachtet nach innen zulaufend ausgebildet. Die konischen Flächen der Greifbacken sind relativ zueinander in einem solchen Abstand angeordnet, dass sie mit den entsprechenden konischen Flächen auf der Aussenwand der Gelenkkomponente von aussen her in Eingriff bringbar sind, wodurch diese zwischen den Greifbacken eingespannt werden kann. Dieses Ausführungsbeispiel zeichnet sich dadurch aus, dass auf die eingespannte Komponente, insbesondere eine Hüftgelenkpfanne, grosse Kräfte (bzw. Momente) übertragen werden können.

Bei einem weiteren Ausführungsbeispiel ist der Stab hohlzylindrisch ausgebildet und die Greifeinrichtung umfasst ein zylindrisches Element, welches im Innenraum des hohlzylindrischen Stabs relativ zu den Greifbacken in distaler bzw. proximaler Richtung verschiebbar angeordnet ist. In diesem zylindrischen Element ist eine quer zu Längsachse des Stabs verlaufende umlaufende Nut vorgesehen, in die ein Fortsatz der jeweiligen Greifbacke hineinragt. Durch eine Bewegung des zylindrischen Elements in distaler bzw. proximaler Richtung können die schwenkbar gelagerten Greifbacken geöffnet bzw. geschlossen werden, wodurch eine zwischen den Greifbacken angeordnete Pfanne freigegeben bzw. gegriffen wird. Dieses Ausführungsbeispiel zeichnet sich durch seinen einfachen konstruktiven Aufbau und die zuverlässige Art und Weise der Betätigung der Greifbacken (durch Bewegen des zylindrischen Elements) aus.

Bei einer Weiterbildung dieses Ausführungsbeispiels ist das zylindrische Element am distalen Ende einer Stange angeordnet, welche am proximalen Ende aus dem hohlzylindrischen Stab herausragt und an welcher dort ein Betätigungsorgan angeordnet ist. Mit Hilfe des Betätigungsorgans an der Stange kann also auf einfache Weise das zylindrische Element in distaler bzw. proximaler Richtung bewegt werden (und somit können die Greifbacken auf einfache Weise geöffnet oder geschlossen werden) und die Pfanne zwischen den Greifbacken eingespannt werden.

Dieses Ausführungsbeispiel kann derart weitergebildet sein, dass die Stange an ihrem distalen Ende mit dem zylindrischen Element verschraubbar ist, und dass das zylindrischen Element mittels einer Drehsicherung gegen eine Verdrehung relativ zu dem hohlzylindrischen Stab gesichert ist. Durch die Verschraubung der Stange am distalen Ende mit dem gegen Verdrehung relativ zum Stab gesicherten zylindrischen Element ist ein einfaches Öffnen und Schliessen der Greifbacken möglich. Dreht man nämlich an der Stange, so schraubt sich das Aussengewinde der Stange in das Innengewinde des zylindrischen Elements hinein bzw. aus diesem heraus, wodurch das zylindrische Element - durch die Steigung des Gewindes bedingt - in proximaler Richtung bzw. in distaler Richtung verschoben wird, da es sich ja wegen der Drehsicherung nicht relativ zum Stab verdrehen kann. Auf diese Art und Weise können beispielsweise beim Greifen der Pfanne die Greifbacken die Pfanne greifen und zwischen den Greifbacken einspannen. Dies ist auf besonders einfache und bequeme Weise möglich. Danach kann das Handhabungsinstrument mitsamt der Pfanne z.B. mit einer Ratsche verbunden werden, mit deren Hilfe dann ein Eindrehen der Pfanne in den vorbereiteten Beckenknochen (oder auch ein Herausdrehen aus demselben) möglich ist.

Die Drehsicherung des zylindrischen Elements gegenüber dem hohlzylindrischen Stab kann beispielsweise so ausgebildet sein, dass der hohlzylindrische Stab eine sich quer zur Längsachse durch den Stab hindurch erstreckende Bohrung aufweist, in welcher ein Stift fest eingepasst, z.B. eingepresst, ist. Das zylindrische Element weist ebenfalls eine sich quer zur Längsachse des Stabs durch das zylindrische Element hindurch sich erstreckende Bohrung auf, nämlich eine Langlochbohrung, durch die hindurch sich der in der Bohrung des Stabs fest eingepasste Stift erstreckt. Bei der Bohrung in dem zylindrischen Element handelt es sich deshalb um eine Langlochbohrung, weil das zylindrische Element ja relativ zum Stab in distaler bzw. proximaler Richtung verschiebbar sein muss, um die Greifbacken zu öffnen und zu schliessen. Auch diese Ausführung zeichnet sich durch konstruktive Einfachheit aus.

Vorzugsweise weist bei all diesen Ausführungsbeispielen die Greifeinrichtung mindestens drei, insbesondere genau drei, Greifbacken auf, die in Umfangsrichtung betrachtet jeweils gleichmässig voneinander beabstandet sind. Dadurch erfolgt eine gleichmässig verteilte Belastung der Pfanne und gleichzeitig eine Zentrierung der Pfanne.

Weiterhin vorteilhaft ist es bei all diesen Ausführungsbeispielen, wenn die Greifeinrichtung zwischen benachbarten Greifbacken im wesentlichen ausgespart ist. Dann kann der Chirurg bzw. Orthopäde nämlich beim Eindrehen der Pfanne in den vorbereiteten Beckenknochen am Instrument vorbei durch eine Bohrung am Pol der Pfanne beobachten, ob die Pfanne bereits so weit in den Beckenknochen eingedreht ist, dass der Boden der Pfanne bereits am Boden der im Beckenknochen vorbereiteten Kavität aufsitzt.

Weitere vorteilhafte Ausgestaltungen ergeben sich aus der nachfolgenden Beschreibung der Zeichnung. In dieser zeigen, teilweise schematisch und/oder im Schnitt:
- Fig. 1: ein Ausführungsbeispiel des erfindungsgemässen Handhabungsinstruments (perspektivisch),
- Fig. 2: das Handhabungsinstrument gemäss Fig. 1, jedoch mit einer eingespannten Hüftgelenkpfanne,
- Fig. 3: das Handhabungsinstrument gemäss Fig. 1, in Schnittdarstellung
und
- Fig. 4: das Handhabungsinstrument mit eingespannter Hüftgelenkpfannge gemäss Fig. 2, in Schnittdarstellung.

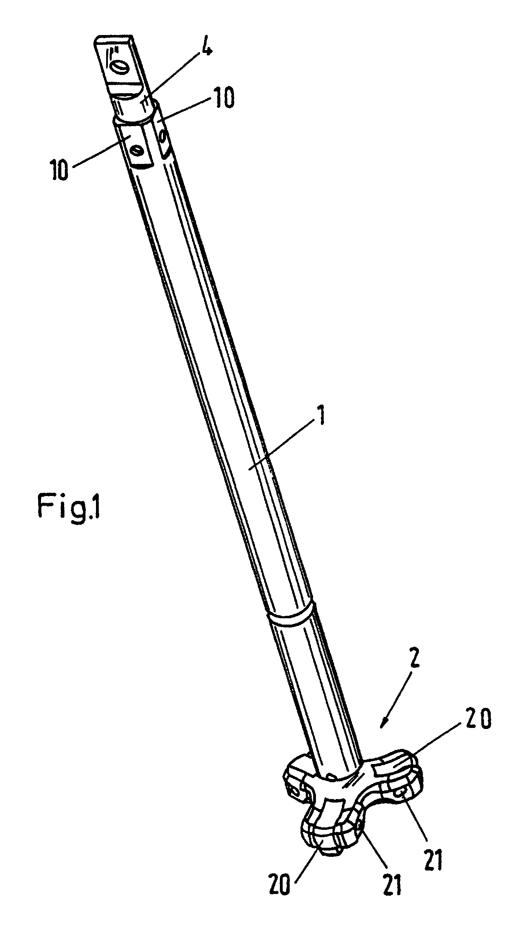

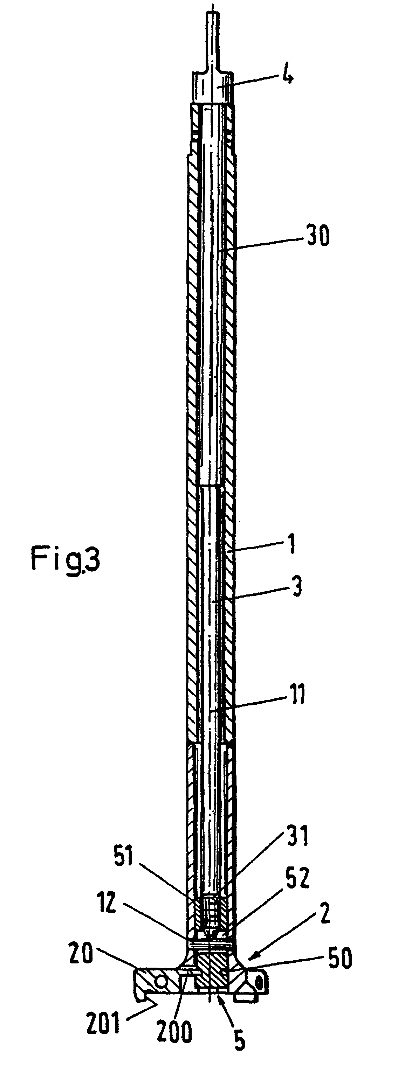

In dem in Fig. 1 perspektivisch dargestellten Ausführungsbeispiel eines erfindungsgemässen Handhabungsinstruments erkennt man einen hohlzylindrischen Stab 1, an dessen distalem Ende eine Greifeinrichtung 2 zum Greifen und Freigeben einer Gelenkkomponente, insbesondere einer Hüftgelenkpfanne, angeordnet ist. Die Greifeinrichtung weist drei in Umfangsrichtung gleichmässig beabstandete (um einen Winkel von jeweils 120° gegeneinander versetzte) Greifbacken 20 auf, die jeweils um einen Stift 21 herum schwenkbar gelagert sind. Am proximalen Ende des Stabs 1 ist sind vier abgeflachte Flächen 10 zu erkennen, die bei eingespannter Pfanne z.B. mit einer Ratsche in Eingriff gebracht werden können. Im Innenraum des hohlzylindrischen Stabs 1 ist eine Stange 3 (hier nicht dargestellt, siehe z.B. Fig. 3) angeordnet, an deren proximalem Ende ein Betätigungsorgan 4 zu erkennen ist.

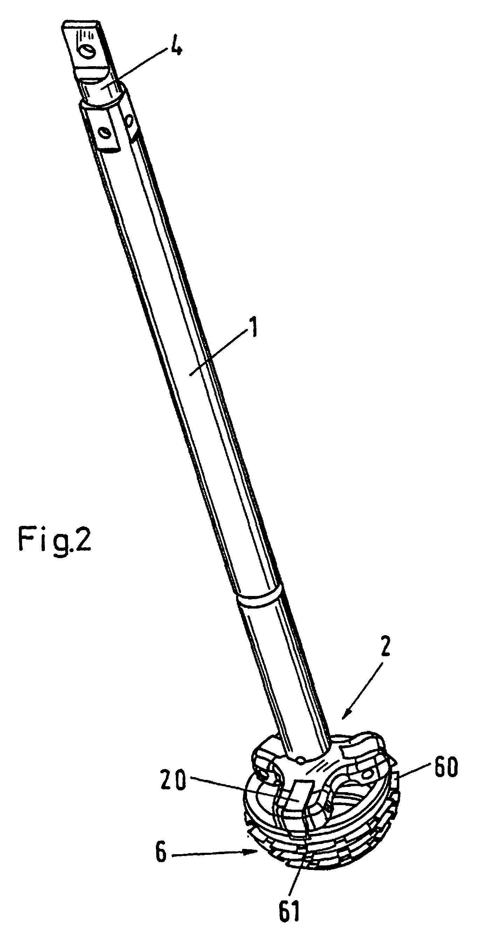

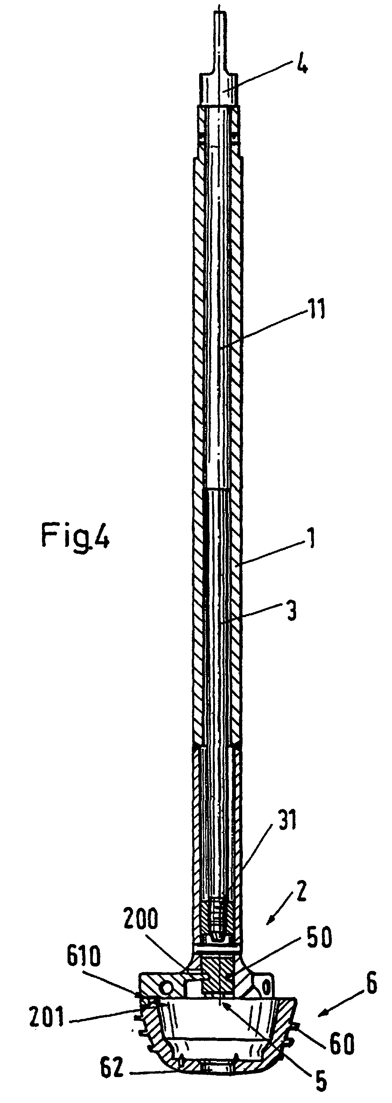

Fig. 2 zeigt im wesentlichen das Ausführungsbeispiel aus Fig. 1, jedoch mit einer eingespannten Hüftgelenkpfanne 6 bzw. der Aussenschale einer solchen Hüftgelenkpfanne. Man erkennt auf der Aussenwand einzelne Stücke 60 eines Gewindes, welches in einen vorbereiteten Beckenknochen eingedreht werden kann. Im Bereich des äquatorialen Rands der Pfanne 6 sind Vertiefungen 61 vorgesehen, welche in distaler Richtung betrachtet konisch nach innen zulaufende Flächen aufweisen. Diese konisch nach innen zulaufenden Flächen können gestrahlt sein, damit sich beim Einspannen keine Diese konisch nach innen zulaufenden Flächen sind hier nicht zu erkennen, da die Greifbacken 20 bzw. deren entsprechenden konischen Flächen (hier ebenfalls nicht zu erkennen, siehe Fig. 3 bzw. Fig. 4) sich mit ihnen in Eingriff befinden.

In Fig. 3 ist das Ausführungsbeispiel des Handhabungsinstruments der Fig. 1 im Schnitt dargestellt. Aus dieser Darstellung kann man die im Innenraum des hohlzylindrischen Stabs 1 angeordnete Stange 3 erkennen, die bei der Erläuterung der Fig. 1 bereits angesprochen worden ist. Die Stange ist an ihrem proximalen Ende (in Fig. 3 also oben) mit dem Betätigungsorgan 4 verschraubbar. Im proximalen Bereich 30 entspricht der Aussendurchmesser der Stange 3 dem Innendurchmesser des hohlzylindrischen Stabs 1, die Stange 3 ist hier also quasi im Stab 1 gelagert. Am distalen Ende der Stange 3 ist ihr Aussendurchmesser jedoch deutlich geringer als der Innendurchmesser des hohlzylindrischen Stabs 1. Dort ist die Stange 3 mit einem Aussengewinde 31 versehen, welches in das Innengewinde 51 eines zylindrischen Elements 5 eingeschraubt ist.

Das zylindrische Element 5 weist eine quer zur Längsachse 11 des Stabs 1 angeordnete, umlaufende Nut 50 auf. In diese Nut 50 ragt ein Fortsatz 200 der jeweiligen Greifbacke 20 hinein. Das zylindrische Element 5 weist ferner eine Langlochbohrung 52 auf. Ebenso weist der Stab 1 an dieser Stelle eine Bohrung auf, in welcher ein Stift 12 eingepasst, z.B. eingepresst ist. Dieser Stift 12 erstreckt sich auch durch die Langlochbohrung 52 des zylindrischen Elements 5 hindurch. Weiterhin erkennt man in Fig. 3 die konische Fläche 201, welche in distaler Richtung nach innen zulaufend ausgebildet ist.

Die Funktionsweise des Handhabungsinstruments ist nun wie folgt. Dabei wird als Beispiel ein Vorgang betrachtet, bei welchem eine künstliche Hüftgelenkpfanne zwischen den Greifbacken 20 eingespannt wird. Zunächst wird das Betätigungsorgan 4 in Öffnungsrichtung gedreht. Dabei wird die Stange 3 ebenfalls gedreht. Infolgedessen bewegt sich kann die Stange 3 mitsamt dem an ihrem distalen Ende aufgeschraubten zylindrischen Element 5 geringfügig in distaler Richtung bewegt werden. Durch die Verschiebung des zylindrischen Elements 5 in distaler Richtung werden die in die Nut 50 hineinragenden Fortsätze 200 der Greifbacken 20 ebenfalls in distaler Richtung bewegt. Da die Greifbacken aber um die Stifte 21 herum schwenkbar gelagert sind und diese Stifte 21 ortsfest angeordnet sind, erfolgt eine entsprechende Verschwenkung der Greifbacken 20, sie befinden sich dann in geöffneter Stellung.

Nun wird das Handhabungsinstrument auf die zu greifende künstliche Gelenkpfanne aufgesetzt, und zwar derart, dass die konischen Flächen 201 in geringfügigem Abstand gegenüber den konischen Flächen 610 in den Vertiefungen 61 im äquatorialen Bereich der Pfanne zu liegen kommen (siehe Fig. 2 bzw. Fig. 4). Das Aufsetzen kann derart erfolgen, dass die Pfanne 6 auf einem sterilen Tuch auf dem Instrumententisch liegt, oder alternativ kann Operationspersonal mit einer Hand die Pfanne 6 halten, mit der anderen Hand das Handhabungsinstrument auf die Pfanne 6 aufsetzen, und dann die Hand, mit der die Pfanne 6 gehalten wird, um die Greifeinrichtung 2 herum schliessen. Dadurch wird die andere Hand wieder frei.

Nach dem Aufsetzen des Handhabungsinstruments auf die Pfanne 6 wird das Betätigungsorgan 4 in Schliessrichtung gedreht. Dadurch wird auch die Stange 3 gedreht, was zur Folge hat, dass sich das zylindrische Element 5 in dem hohlzylindrischen Stab 1 aufwärts bewegt, weil der Stift 12 ja eine Drehsicherung bildet, also ein Verdrehen des zylindrischen Elements 5 gegenüber dem Stab 1 verhindert. Da aber mittels des Betätigungsorgans 4 an der Stange 3 gedreht wird, muss eine Verdrehung der Stange 3 gegenüber dem drehfest angeordneten zylindrischen Element 5 stattfinden. Infolgedessen bewegt sich das zylindrische Element nach oben. Dabei wird auch klar, warum die Bohrung in dem zylindrischen Element 5 als Langlochbohrung 52 ausgebildet ist, weil nämlich beim Betätigen des Betätigungsorgans 4 eine Verschiebung des zylindrischen Elements 5 relativ zum Stab 1 in proximaler (bzw. beim Öffnen in distaler) Richtung erfolgt, der Stift 12 aber ortsfest angeordnet ist. Die Bewegung des zylindrischen Elements 5 in proximaler Richtung hat eine entsprechende Schwenkbewegung der Greifbacken 20 um die Stifte 21 herum zur Folge, wodurch die konischen Flächen 201 der Greifbacken 20 fest and den konischen Flächen 610 in den Vertiefungen 61 der Pfanne 6 zu liegen kommen. Liegen die konischen Flächen fest aneinander an, so ist die Pfanne 6 fest zwischen den Greifbacken eingespannt. Dieser Zustand ist in Fig. 4 zu erkennen, in welcher eine Pfanne 6 fest zwischen den Greifbacken 20 eingespannt ist.

Wie in Fig. 4 zu erkennen ist, weist die Pfanne 6 an ihrem Pol eine Bohrung 62 auf. Dies ist deshalb erwähnenswert, weil bei dem gezeigten Ausführungsbeispiel des Handhabungsinstruments die Greifeinrichtung 2 zwischen jeweils benachbarten Greifbacken im wesentlichen vollständig ausgespart ist. Dies ermöglicht dem Chirurgen bzw. Orthopäden, beim Eindrehen der Pfanne 6 am Instrument vorbei, nämlich durch die ausgesparten Bereiche, zu verfolgen, wann die Pfanne 6 soweit in den vorbereiteten Beckenknochen eingedreht ist, dass der Boden der Pfanne in der vorbereiteten Kavität aufsitzt. Dies kann er nämlich durch die Bohrung 62 im Boden der Pfanne erkennen, jedoch nur wegen der ausgesparten Bereiche zwischen den benachbarten Greifbacken.

Das Eindrehen der Pfanne 6 in den Beckenknochen kann, wie bereits erwähnt, derart erfolgen, dass das Handhabungsinstrument mit eingespannter Pfanne 6 z.B. mit einer Ratsche verbunden wird. Zu diesem Zweck sind am proximalen Ende des Stabs 1 die nach Muster eines Vierkants vorgesehenen abgeflachten Flächen 10 vorgesehen.

Zur Sterilisation des Handhabungsinstruments nach einer Operation wird mittels des Betätigungsorgans 4 die Stange 3 aus dem zylindrischen Element 5 vollständig herausgeschraubt und die Stange 3 wird in proximaler Richtung aus dem hohlzylindrischen Stab 1 herausgezogen. Die einzelnen Teile können dann in den Sterilisator gelegt werden. Da das zylindrische Element 5 ja in dem hohlzylindrischen Stab 1 gleitend verschiebbar ist, muss das Instrument nicht weiter zerlegt werden. Dennoch kann eine vollständige Sterilisation des Handhabungsinstruments erfolgen, wobei der Aufwand für die Zerlegung denkbar gering ist.

Es ist ohne weiteres ersichtlich, dass das erfindungsgemässe Handhabungsinstrument ganz allgemein für die Handhabung von Gelenkkomponenten geeignet ist, also beispielsweise auch für Gelenkkomponenten des Schultergelenks oder anderer Gelenke.

## Patentansprüche

1. System aus einer künstlichen Gelenkkomponente, insbesondere einer künstlichen Hüftgelenkpfanne (6), und einem Instrument zur Handhabung der künstlichen Gelenkkomponente, wobei das Handhabungsinstrument einen Stab (1) umfasst, an dessen distalem Ende eine betätigbare Greifeinrichtung (2) zum Greifen und Freigeben der Gelenkkomponente angeordnet ist, welche Greifeinrichtung (2) mindestens zwei Greifbacken (20) umfasst,
**dadurch gekennzeichnet,**
**dass** die Gelenkkomponente bei bestimmungsgemäßer, zum Einspannen der Gelenkkomponente dienender Einwirkung des Handhabungsinstrumentes formstabil und insbesondere als auf der Außenwand mit einem Gewinde versehene Schraubpfanne ausgebildet ist,
**dass** an den Greifbacken (20) jeweils eine konische Fläche (201) vorgesehen ist, die zum Einspannen der Gelenkkomponente mit einer an der Gelenkkomponente entsprechend vorgesehenen konischen Fläche (610) in Eingriff bringbar ist,
**dass** die konische Fläche (201) auf der Innenseite der jeweiligen Greifbacke (20) vorgesehen und in distaler Richtung betrachtet nach innen zulaufend ausgebildet ist, und
**dass** die konischen Flächen (201) der Greifbacken (20) relativ zueinander in einem solchen Abstand angeordnet sind, dass sie zum Einspannen der Gelenkkomponente mit einer auf der Außenwand der Gelenkkomponente entsprechend vorgesehenen, ebenfalls in distaler Richtung betrachtet nach innen zulaufenden konischen Fläche (610) von außen her in Eingriff bringbar sind.

2. System nach Anspruch 1, **dadurch gekennzeichnet, dass** die Greifbacken (20) schwenkbar gelagert sind.

3. System nach einem der vorangehenden Ansprüche, **dadurch gekennzeichnet, dass** der Stab (1) hohlzylindrisch ausgebildet ist und dass die Greifeinrichtung (2) ein zylindrisches Element (5) umfasst, welches im Innenraum des hohlzylindrischen Stabs (1) relativ zu den Greifbacken (20) in distaler bzw. proximaler Richtung verschiebbar angeordnet ist, und **dass** in diesem zylindrischen Element (5) eine quer zur Längsachse (11) des Stabs (1) verlaufende umlaufende Nut (50) vorgesehen ist, in die ein Fortsatz (200) der jeweiligen Greifbacke (20) hineinragt.

4. System nach Anspruch 3, **dadurch gekennzeichnet, dass** das in dem hohlzylindrischen Stab (1) angeordnete zylindrische Element (5) am distalen Ende einer Stange (3) angeordnet ist, welche am proximalen Ende aus dem hohlzylindrischen Stab (1) herausragt und an welcher dort ein Betätigungsorgan (4) angeordnet ist.

5. System nach Anspruch 4, **dadurch gekennzeichnet, dass** die Stange (3) an ihrem distalen Ende mit dem zylindrischen Element (5) verschraubbar ist, und **dass** das zylindrische Element mittels einer Drehsicherung (12,52) gegen eine Verdrehung relativ zu dem hohlzylindrischen Stab gesichert ist.

6. System nach Anspruch 5, **dadurch gekennzeichnet, dass** die Drehsicherung derart ausgebildet ist, dass der hohlzylindrische Stab (1) eine sich quer zu seiner Längsachse (11) durch den Stab hindurch erstreckende Bohrung aufweist, in welcher ein Stift (12) fest eingepasst ist, und **dass** das zylindrische Element (5) eine sich ebenfalls quer zur Längsachse (11) des Stabs (1) durch das zylindrische Element (5) hindurch erstreckende Langlochbohrung (52) aufweist, durch die hindurch sich der in der Bohrung des Stabs (1) fest eingepasst Stift (12) erstreckt.

7. System nach einem der vorangehenden Ansprüche, **dadurch gekennzeichnet, dass** die Greifeinrichtung (2) mindstens drei, insbesondere genau drei, Greifbacken (20) aufweist, die in Umfangsrichtung betrachtet jeweils gleichmässig voneinander beabstandet sind.

8. System nach einem der vorangehenden Ansprüche, **dadurch gekennzeichnet, dass** die Greifeinrichtung (2) zwischen benachbarten Greifbacken (20) im wesentlichen vollständig ausgespart ist.

## Claims

1. A system comprising an artificial joint component, in particular an artificial hip joint socket (6), and an instrument for the manipulation of the artificial joint component, with the manipulation instrument comprising a bar (1), at the distal end of which an actuatable gripper apparatus (2) for the gripping and releasing of the joint component is arranged, said gripper apparatus (2) comprising at least two gripper jaws (20), **characterised**
**in that** the joint component is formed in a stable shape when used as intended for the influencing of the manipulation instrument serving to clamp the joint component, and is in particular formed as a screw socket provided with a thread at the outer wall;
**in that** a conical surface (201) is in each case provided at the gripper jaws (20) which can be brought into engagement with a conical surface (610) correspondingly provided at the joint component for the clamping of the joint component;
**in that** the conical surface (201) is provided on the inner surface of the respective gripper jaw (20) and is formed to taper inwardly when viewed in the distal direction; and
and **in that** the conical surfaces (201) of the gripper jaws (20) are arranged relative to one another at a spacing such that, when clamping the joint component, they can be brought from the outside into engagement with a conical surface (610) correspondingly provided at the outer wall of the joint component and likewise tapering inwardly when viewed in the distal direction

2. A system in accordance with claim 1, **characterised in that** the gripper jaws (20) are pivotally journalled.

3. A system in accordance with any one of the preceding claims **characterised in that** the bar (1) is formed as a hollow cylinder; and **in that** the gripper apparatus (2) comprises a cylindrical element (5) which is arranged displaceably in the distal and proximal directions relative to the gripper jaws (20) in the inner space of the hollow cylindrical bar (1); and **in that** a circumferential groove (50), which extends transversely to the longitudinal axis (11) of the bar (1), is provided in this cylindrical element (5) into which an extension (200) of the respective gripper jaw (20) projects.

4. A system in accordance with claim 3 **characterised in that** the cylindrical element (5) arranged in the hollow cylindrical bar (1) is arranged at the distal end of a rod (3) which projects out of the hollow cylindrical bar (1) at the proximal end and at which an actuation member (4) is arranged there.

5. A system in accordance with claim 4 **characterised in that**, at its distal end, the rod (3) can be screwed to the cylindrical element (5); and **in that** the cylindrical element is secured against rotation relative to the hollow cylindrical bar by a means providing security against rotation (12, 52).

6. A system in accordance with claim 5 **characterised in that** the security against rotation is formed such that the hollow cylindrical bar (1) has a bore which extends transversely to its longitudinal axis (11) and through the bar and in which a pin (12) is firmly fitted; and **in that** the cylindrical element (5) has an elongate bore (52) which likewise extends transversely to the longitudinal axis (11) of the bar (1) and through the cylindrical element (5) and through which the pin (12) extends which is firmly fitted in the bore of the bar (1).

7. A system in accordance with any one of the preceding claims **characterised in that** the gripper apparatus (2) has at least three, in particular precisely three, gripper jaws (20) which are in each case uniformly spaced with respect to one another when viewed in the peripheral direction.

8. A system in accordance with any one of the preceding claims **characterised in that** the gripper apparatus (2) is substantially completely cut away between adjacent gripper jaws (20).

## Revendications

1. Système constitué d'un composant articulaire artificiel, notamment d'une coque acétabulaire artificielle (6), et d'un instrument pour manipuler le composant articulaire artificiel, où l'instrument de manipulation comporte une tige (1) à l'extrémité distale de laquelle est disposée une installation de préhension actionnable (2) pour prendre et libérer le composant articulaire, cette installation de préhension (2) comprenant au moins deux mâchoires de préhension (20),
**caractérisé en ce que** le composant articulaire, sous l'action conforme au but de l'instrument de manipulation, servant à serrer le composant articulaire, est réalisé en une forme stable et notamment comme coque à visser pourvue sur la paroi extérieure d'un filetage,
**en ce qu'**il est prévu aux mâchoires de préhension (20) respectivement une face conique (201) qui, pour le serrage du composant articulaire, peut être mise en prise avec une face conique (610) prévue d'une manière correspondante au composant articulaire,
**en ce que** la surface conique (201) est prévue sur le côté intérieur de la mâchoire de préhension respective (20) et, vue dans la direction distale, est réalisée pour diminuer vers l'intérieur, et
**en ce que** les surfaces coniques (201) des mâchoires de préhension (20) sont disposées l'une par rapport à l'autre à un tel écart qu'elles peuvent être mises en prise, pour serrer le composant articulaire, depuis l'extérieur avec une face conique (610) prévue d'une manière correspondante sur la paroi extérieure du composant articulaire, s'étendant également vers l'intérieur, en regardant dans la direction distale.

2. Système selon la revendication 1, **caractérisé en ce que** les mâchoires de préhension (20) sont logées d'une manière pivotante.

3. Système selon l'une des revendications précédentes, **caractérisé en ce que** la tige (1) est réalisée en cylindre creux, et **en ce que** l'installation de préhension (2) comporte un élément cylindrique (5) qui est disposé d'une manière déplaçable dans l'espace intérieur de la tige cylindrique creuse (1) relativement aux mâchoires de préhension (20) dans la direction distale respectivement proximale, et **en ce qu'**il est prévu dans cet élément cylindrique (5) une rainure (50) s'étendant tout autour, s'étendant transversalement à l'axe longitudinal (11) de la tige (1), dans laquelle fait saillie un prolongement (200) de la mâchoire de préhension respective (20).

4. Système selon la revendication 3, **caractérisé en ce que** l'élément cylindrique (5) disposé dans la tige cylindrique creuse (1) est disposé à l'extrémité distale d'une tige (3) qui dépasse à l'extrémité proximale de la tige cylindrique creuse (1) et à laquelle y est disposé un organe d'actionnement (4).

5. Système selon la revendication 4, **caractérisé en ce que** la tige (3) peut être assemblée par vissage à son extrémité distale avec l'élément cylindrique (5), et **en ce que** l'élément cylindrique est assuré au moyen d'une sécurité de rotation (12, 52) à l'encontre d'une rotation relativement à la tige cylindrique creuse.

6. Système selon la revendication 5, **caractérisé en ce que** la sécurité de rotation est réalisée de façon que la tige cylindrique creuse (1) présente un perçage s'étendant transversalement à son axe longitudinal (11) à travers la tige, dans lequel une cheville (12) est insérée solidement, et **en ce que** l'élément cylindrique (5) présente un perçage de trou oblong (52) s'étendant également transversalement à l'axe longitudinal (11) de la tige (1) à travers l'élément cylindrique (5), à travers lequel s'étend la cheville (12) insérée solidement dans le perçage de la tige (1).

7. Système selon l'une des revendications précédentes, **caractérisé en ce que** l'installation de préhension (2) présente au moins trois, en particulier exactement trois, mâchoires de préhension (20) qui, vues dans la direction périphérique, sont espacées d'une manière équidistante les unes des autres.

8. Système selon l'une des revendications précédentes, **caractérisé en ce que** l'installation de préhension (2) est sensiblement complètement évidée entre des mâchoires de préhension avoisinantes (20).
